# DEMANDE DE BREVET EUROPEEN

(11) **EP 4 821 527 A1**
(43) Date de publication de la demande: **14.10.2026**
(21) Numéro de dépôt: 26161094.3
(22) Date de dépôt: 26.02.2026
(51) Int. Cl.: A61M 16/00, A61M 16/12, A61M 16/20

(54) **APPAREIL DE DÉLIVRANCE DE NO INTÉGRANT DES MOYENS DE PURGE DE CONDUIT FLEXIBLE**

(30) Priorité: 08.04.2025 FR 2503723
(71) Demandeur: INOSYSTEMS, 92160 Antony (FR)
(72) Inventeur: MARCHAL, Frederic, 92160 Antony (FR); PROUVEZ, Nathan, 92160 Antony (FR); BLANDIN, Yann, 92160 Antony (FR); BOULANGER, Thierry, 92160 Antony (FR)
(74) Mandataire: Air Liquide

(57) **Abrégé**

L'invention concerne un appareil de délivrance de NO (1) comprenant un circuit de gaz principal (200) avec une entrée (2) de NO configurée pour y raccorder un conduit flexible (12) amenant le gaz contenant du NO. Des premiers moyens de contrôle de flux (210) contrôlent la circulation du flux de gaz dans le circuit de gaz principal (200) auquel est relié un circuit de purge (250) avec une sortie de purge (6) en communication avec l'atmosphère. Des seconds moyens de contrôle de flux (252) du circuit de purge contrôlent la circulation de gaz vers la sortie de purge. Des moyens de pilotage (205) contrôlent les moyens de contrôle de flux (210 ; 252) pour opérer une purge du conduit flexible, en réponse à un appui par l'utilisateur d'une touche virtuelle s'affichant sur un écran tactile.

## Description

L'invention concerne un appareil de délivrance de NO et une installation de fourniture d'un mélange gazeux à base de NO à un patient comprenant un tel appareil de délivrance de NO, permettant à l'utilisateur d'opérer une purge d'un conduit flexible raccordé au circuit de gaz principal de l'appareil de délivrance de NO, lorsque le récipient de gaz alimentant ledit conduit flexible est vide ou quasi-vide et doit être remplacé par un récipient plein.

Le monoxyde d'azote inhalé, i.e. NO ou NOi, est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP560928 ou EP1516639.

Pour mettre en œuvre une thérapie par NO inhalé, on utilise une installation de fourniture de gaz, aussi appelée installation d'administration de gaz ou de NO, comprenant un appareil de délivrance de NO et un ventilateur médical, c'est-à-dire un appareil d'assistance respiratoire, alimentant chacun un circuit patient.

L'appareil de délivrance de NO permet d'injecter un mélange gazeux à base de NO, typiquement un mélange NO/azote, dans le circuit patient alimenté par ailleurs en un flux gazeux contenant de l'oxygène (au moins environ 20% vol.), tel de l'air ou un mélange oxygène/azote (O₂/N₂), fourni par le ventilateur médical. Le circuit patient comprend en général un ou plusieurs conduits flexibles reliés fluidiquement à une interface respiratoire, telle une sonde d'intubation trachéale ou analogue, servant à délivrer au patient à traiter, un mélange gazeux thérapeutique contenant une quantité ou dose donnée de NO, c'est-à-dire une posologie, typiquement entre 5 et 40 ppmv de NO, ou une autre posologie choisie par le médecin.

De telles installations de fourniture de gaz sont décrites par exemple par EP3821929, EP4209243, EP4241817, EP4241812 et EP4295882.

Le NO gazeux, typiquement un mélange gazeux NO/azote, alimentant l'appareil de délivrance de NO provient généralement de récipients de gaz, simplement appelés « bouteilles de NO », reliés fluidiquement à l'appareil par des conduits ou tuyaux flexibles servant à véhiculer le gaz.

Raccorder fluidiquement l'appareil de délivrance de NO à deux bouteilles de NO (i.e. de mélange NO/azote), au moyen de tuyaux flexibles adaptés, permet d'éviter toute interruption de traitement du patient puisque lorsqu'une bouteille de NO est vide, l'autre bouteille de NO qui est pleine peut alors prendre le relais et fournir le mélange NO/N₂ à l'appareil de délivrance de NO.

Habituellement, la liaison entre chaque bouteille de NO et l'appareil de délivrance de NO est assurée par un conduit ou tuyau flexible ou analogue qui vient se raccorder à la sortie d'un dispositif réducteur de pression externe, c'est-à-dire un détendeur de gaz, venant se fixer au raccord de sortie du robinet de distribution de gaz équipant la bouteille de NO. Le détendeur de gaz permet de réduire la pression du gaz sortant de la bouteille qui peut atteindre plus de 150 bars abs (bouteille pleine) jusqu'à une pression inférieure donnée, par exemple entre 3 et 10 bars abs.

Pour remplacer une bouteille de NO vide (ou quasiment vide) par une nouvelle bouteille de NO pleine, il faut pouvoir déconnecter la bouteille de NO vide, i.e. devant être remplacée, du conduit ou tuyau flexible qui la relie fluidiquement à l'appareil de délivrance de NO, en particulier du détendeur de gaz auquel vient de fixer le conduit flexible.

Or, cela n'est possible tant que l'utilisateur n'a pas purgé la pression résiduelle de NO gazeux (i.e. mélange NO/N₂), qui peut être de plusieurs bars, par exemple de 3 à 7 bars, qui est piégée dans le tuyau flexible entre l'appareil de délivrance de NO et le détendeur et/ou la bouteille de NO.

Pour ce faire, l'utilisateur doit d'abord débrancher le conduit ou tuyau flexible du côté de l'appareil de délivrance de NO, en particulier du détendeur, puis purger ledit tuyau flexible à l'aide d'un outillage dédié, pour évacuer la pression résiduelle de NO gazeux qui s'y trouve et ce, afin de pouvoir ensuite dévisser le détendeur de la bouteille vide avant de le remonter sur la bouteille de NO pleine de remplacement.

On comprend qu'une telle opération est fastidieuse, consommatrice de temps et peut présenter des risques pour l'utilisateur du fait de la pression résiduelle régnant dans le tuyau flexible. D'ailleurs, le conduit étant soumis à une pression interne résiduelle de plusieurs bars, par exemple de 3 à 7 bars, il très difficile, voire parfois quasi-impossible, de le démonter du dispositif détendeur.

EP4241817 et EP2522384 enseigne de devoir opérer une purge régulière de l'appareil de délivrance de NO et US6125846 précise que la purge peut être automatique ou manuelle, c'est-à-dire lancée par un opérateur. Cependant, ces documents ne détaillent pas l'opération de purge, ni celle de remplacement d'une bouteille de NO vide par une bouteille neuve, i.e. pleine.

Un problème est dès lors de pouvoir simplifier les opérations de remplacement d'une bouteille de NO vide afin d'éviter à l'utilisateur de devoir réaliser des opérations de déconnexion et reconnexion fastidieuses, et par ailleurs de purge manuelle du tuyau flexible, lors du remplacement d'une bouteille de NO vide alimentant un appareil de délivrance de NO raccordé de préférence à deux (ou plus) bouteilles de NO, lequel fait partie d'une installation de fourniture d'un mélange gazeux contenant du NO à un patient.

Une solution selon l'invention concerne un appareil de délivrance de NO pour fournir un gaz contenant du NO, typiquement un mélange gazeux NO/N₂, comprenant :
- un circuit de gaz principal pour véhiculer le gaz contenant du NO entre au moins une entrée de NO et au moins une sortie de NO,
- ladite au moins une entrée de NO configurée pour permettre d'y raccorder fluidiquement un conduit flexible de manière à alimenter le circuit de gaz principal avec le gaz contenant du NO amené par ledit conduit flexible,
- des premiers moyens de contrôle de flux agencés sur ledit circuit de gaz principal, configurés pour contrôler la circulation du flux de gaz contenant du NO dans ledit circuit de gaz principal,
- des moyens de pilotage à microprocesseur,
- un circuit de purge raccordé fluidiquement au circuit de gaz principal entre ladite au moins une entrée de NO et les premiers moyens de contrôle de flux, ledit circuit de purge comprenant une sortie de purge en communication fluidique avec l'atmosphère,
- des seconds moyens de contrôle de flux agencés sur ledit circuit de purge, configurés pour contrôler la circulation de gaz dans ledit circuit de purge en direction de la sortie de purge, et
- des moyens de déclenchement de purge, actionnables par l'utilisateur.

Selon l'invention, l'appareil comprend en outre un écran d'affichage tactile et :
- les moyens de déclenchement de purge comprennent une touche de lancement de purge virtuelle s'affichant sur l'écran d'affichage tactile et
- les moyens de pilotage sont configurés pour piloter les premiers et seconds moyens de contrôle de flux de manière à stopper (i.e. interdire) la circulation de gaz dans au moins une partie du circuit de gaz principal et à permettre (i.e. autoriser) une circulation de gaz dans le circuit de purge, en réponse à un appui digital (i.e. contact du doigt) par l'utilisateur sur la touche virtuelle, i.e. sur les moyens de déclenchement de purge.

Selon le mode de réalisation considéré, l'appareil de délivrance de NO, i.e. de mélange gazeux NO/N₂, selon l'invention peut comprendre l'une ou plusieurs des caractéristiques suivantes :
- les premiers et seconds moyens de contrôle de flux comprennent au moins une vanne, en particulier des électrovannes dites « principales » pilotées par les moyens de pilotage.
- l'écran d'affichage tactile comprend une dalle tactile ou analogue.
- la touche de lancement de purge virtuelle s'affiche sur l'écran d'affichage tactile au sein d'une fenêtre affichée sur l'écran, i.e. une fenêtre dédiée, typiquement une fenêtre de lancement de purge.
- la touche de lancement de purge est configurée pour être activée par un appui ou contact du doigt par l'utilisateur sur une zone donnée de l'écran tactile.
- l'écran tactile est configuré pour détecter le contact du doigt de l'utilisateur sur la zone donnée de l'écran tactile correspondant à la touche de lancement de purge, c'est-à-dire lorsque la touche de lancement de purge est ou a été activée, et transmettre un signal d'activation aux moyens de pilotage.
- les moyens de pilotage sont configurés pour recevoir et traiter le signal d'activation.
- les moyens de pilotage sont configurés pour interpréter ou reconnaitre le signal d'activation comme étant un signal de début de purge.
- les moyens de pilotage sont configurés pour débuter une purge en réponse audit signal de début de purge.
- les moyens de pilotage commandent un début de purge en agissant sur les premiers et seconds moyens de contrôle de flux, en réponse à un actionnement des moyens de déclenchement de purge, c'est-à-dire à un appui digital (e.g. du doigt) sur la touche virtuelle de la part de l'utilisateur.
- le circuit de gaz principal comprend deux tronçons d'entrée agencés en parallèles.
- chaque tronçon d'entrée comprend une entrée de NO permettant d'alimenter le circuit de gaz principal en gaz contenant du NO, typiquement un mélange gazeux NO/N₂.
- les deux tronçons d'entrée se raccordent fluidiquement l'un à l'autre en un site de jonction du circuit de gaz principal.
- le circuit de purge comprend deux tronçons de jonction, agencés en parallèles.
- chaque tronçon de jonction vient se raccorder à l'un des tronçon d'entrée du circuit de gaz principal, en amont des premiers moyens de contrôle de flux agencés sur ledit circuit de gaz principal.
- les deux tronçons de jonction se raccordent fluidiquement l'un à l'autre en un site de réunion du circuit de purge.
- des seconds moyens de contrôle de flux sont agencés sur les deux tronçons de jonction, typiquement électrovannes dites « de purge » aussi pilotées par les moyens de pilotage.
- en réponse à un appui (du doigt) de l'utilisateur sur la touche de lancement de purge virtuelle s'affichant sur l'écran d'affichage tactile, les moyens de pilotage sont configurés pour piloter les électrovannes principales et de purge de manière :
   ▪ à stopper la circulation de gaz dans la partie du circuit de gaz principal située en aval des électrovannes principales en fermant ou maintenant en position de fermeture, les deux électrovannes principales ;
   ▪ à maintenir en position de fermeture l'une des électrovannes de purge ; et
   ▪ à permettre une circulation de gaz dans le circuit de purge en passant l'autre des électrovannes de purge en position d'ouverture pour autoriser une circulation du gaz dans le circuit de purge.
- il comprend deux entrées de NO configurées pour être raccordées chacune à un conduit flexible d'acheminement de gaz, i.e. de mélange NO/N₂.
- la ou les entrées de NO peuvent être portées chacune par un raccord ou connecteur fluidique.

Par ailleurs, selon le mode de réalisation considéré, l'appareil de délivrance de NO, i.e. de mélange gazeux NO/N₂, selon l'invention peut comprendre l'une ou plusieurs des caractéristiques additionnelles suivantes :
- les récipients de NO, i.e. bouteilles de gaz, contiennent un mélange NO/N₂ contenant entre 100 et 5000 ppmv de NO, et de l'azote pour le reste, de préférence entre 100 et 2000 ppmv, typiquement entre 200 et 1000 ppmv.
- il est alimenté en un mélange gazeux formé d'azote et de NO, i.e. mélange binaire.
- le (les) récipient de NO vide contient un mélange NO/N₂ résiduel à une pression préfixée dite pression résiduelle, de préférence inférieure ou égale à 25 bars, par exemple de l'ordre de 25 bars.
- il comprend des moyens de réglage de dose configurés pour permettre à un utilisateur de fixer ou sélectionner une consigne de teneur en NO correspondant à la proportion finale de NO souhaitée dans le mélange gazeux combiné, i.e. une posologie.
- les moyens de réglage de dose font partie d'une IHM (interface homme-machine) ou IGU (interface graphique utilisateur).
- l'écran est du type à affichage en couleurs.
- l'écran digital, i.e. à dalle tactile, fait partie de l'IHM.
- les moyens de réglage de dose comprennent une ou des touches tactiles, actionnables par l'utilisateur, typiquement par appui ou pression digitale (i.e. du doigt).
- la ou les touches tactiles de réglage de dose s'affichent sur l'écran digital.
- la consigne de teneur en NO est comprise entre 1 et 80 ppmv, typiquement entre 5 et 40 ppmv.
- il comprend des moyens de mémorisation, à savoir une mémoire informatique, telle une mémoire flash, une RAM ou analogue.
- les moyens de pilotage comprennent un (micro)contrôleur ou analogue.
- les moyens de pilotage comprennent un (ou des) (micro)processeur agencé sur une (ou des) carte électronique.
- les moyens de pilotage comprennent un (ou des) (micro)processeur mettant en oeuvre un ou des algorithmes, notamment un (ou des) algorithme de pilotage ou de contrôle des vannes ou valves, de traitement des mesures de débit ou de pression....ou autre.
- les moyens de mémorisation sont agencés sur la carte électronique.
- il est alimenté électriquement par une ou des sources de courant électrique, typiquement le secteur (110/220V) et/ou une ou des batteries rechargeables.
- il comprend une coque ou carcasse périphérique.
- les deux tronçons d'entrée, le circuit de gaz principal, le circuit de purge, les premiers et/ou seconds moyens de contrôle de flux sont agencés dans coque ou carcasse périphérique de l'appareil.

L'invention concerne en outre une installation de fourniture d'un mélange gazeux contenant du NO à un patient comprenant :
- au moins un récipient de NO contenant le gaz contenant du NO, de préférence deux récipients de NO, typiquement un mélange gazeux NO/N₂,
- l'appareil de délivrance de NO selon l'invention, et
- au moins un conduit flexible reliant fluidiquement ledit au moins un récipient de NO à l'appareil de délivrance de NO, de préférence chaque récipient de NO est relié à l'appareil de délivrance de NO par un conduit flexible.

Selon le mode de réalisation considéré, l'installation de fourniture de gaz selon l'invention peut comprendre l'une ou plusieurs des caractéristiques suivantes :
- au moins un dispositif de détente, tel un détendeur de gaz, est agencé en aval dudit au moins un récipient de NO.
- ledit au moins un conduit flexible est accordé fluidiquement audit au moins un dispositif de détente.
- elle comprend en outre un ventilateur médical configuré pour fournir un flux de gaz respiratoire contenant de I'O₂ (≥ 20%vol.), tel de l'air ou un mélange O₂/N₂.
- elle comprend en outre un circuit respiratoire auquel sont raccordés fluidiquement le ventilateur médical et l'appareil de délivrance de NO, en particulier à une branche inspiratoire dudit circuit respiratoire.
- un capteur de débit est agencé dans le circuit respiratoire.
- un dispositif d'injection permet d'injecter le flux gazeux contenant du NO (e.g. mélange NO/N₂) dans le flux de gaz respiratoire contenant de l'O₂ (e.g. air ou mélange O₂/N₂) pour obtenir un mélange gazeux combiné, i.e. mélange final, contenant du NO, de l'oxygène et de l'azote.
- le ventilateur médical délivre un gaz respiratoire contenant au moins 20% vol. environ d'oxygène, de préférence au moins 21% vol. environ d'oxygène.
- le ventilateur médical comprend une soufflante motorisée (i.e. turbine, compresseur ou analogue) délivrant le gaz respiratoire, typiquement de l'air ou un mélange oxygène/azote ou, selon un autre mode de réalisation, un circuit de gaz interne comprenant une ou des vannes proportionnelles pour acheminer le gaz et contrôler sa fourniture, notamment son débit. Un tel ventilateur est généralement alimenté en gaz respiratoire par une (ou des) prise murale alimentée en gaz par un réseau de canalisations d'un établissement ou bâtiment hospitalier, typiquement de l'air ou un mélange oxygène/azote.
- le ventilateur médical comprend des moyens ou un dispositif de commande, telle une (ou des) carte de commande électronique. De préférence, les moyens de commande du ventilateur médical pilotent ou commandent la soufflante motorisée ou, selon le cas, les valves proportionnelles du ventilateur médical.
- le ventilateur médical est de type HFO ou comprend une fonction de HFO, c'est-à-dire qu'il est apte à produire des oscillations à haute fréquence.
- chaque récipient de NO contient un mélange gazeux NO/N₂ contenant entre 100 et 2000 ppmv de NO, le reste étant de l'azote (N₂), de préférence entre 100 et 1000 ppmv de NO, par exemple 450 ou 800 ppmv.
- chaque récipient de NO contient un mélange gazeux NO/N₂ conditionné à une pression (mesurée avant début de soutirage) comprise entre 10 et 250 bars, de préférence plus de 50 bars, typiquement à plus de 100 bars.
- chaque récipient de NO est ou comprend une (ou des) bouteille de gaz ayant une contenance comprise entre 0,5 et 50L (équivalent en eau).
- chaque récipient de NO comprend un corps cylindrique en acier ou en alliage d'aluminium.
- chaque récipient de NO est équipé d'un robinet de distribution de gaz dit « simple » (sans détendeur intégré) ou d'un robinet dit « à détendeur intégré » (RDI) intégrant des moyens de détente (i.e. de réduction de pression), de préférence un RDI.
- chaque robinet de distribution de gaz (i.e. simple ou RDI) est protégé par un capotage de protection, par exemple en métal ou polymère.
- chaque récipient de NO est équipé d'un robinet « simple » (i.e. sans détendeur intégré) et un dispositif détendeur de gaz est agencé en aval de chaque robinet.
- les moyens de réduction de pression, i.e. dispositif détendeur de gaz, sont agencés en aval du robinet « simple » de chaque récipient de NO contenant le gaz à base de NO, typiquement un mélange NO/N₂ de manière à opérer une diminution ou réduction de pression du gaz à base de NO, typiquement un mélange NO/N₂, sortant du robinet considéré, i.e. de chaque robinet, et obtenir ainsi le gaz à la pression de détente (PD) souhaitée inférieure à 10 bars.
- le dispositif détendeur est fixé à un robinet (simple) en étant raccordé fluidiquement à la sortie en pression dudit robinet, par exemple par vissage ou analogue.
- les moyens de réduction de pression sont configurés pour réduire la pression du gaz à base de NO, typiquement un mélange NO/N₂, jusqu'à une pression de détente inférieure ou égale à 10 bars, de préférence inférieure à 8 bars, typiquement entre 2 et 7 bars, par exemple entre 5 et 6 bars.
- le ou chaque conduit flexible est configuré pour acheminer le gaz à base de NO après réduction de sa pression jusqu'à la pression de détente inférieure ou égale à 10 bars, c'est-à-dire un gaz détendu.
- le ou chaque conduit flexible comprend un tuyau en polymère.
- le circuit respiratoire comprend une branche inspiratoire et une branche expiratoire, typiquement des conduites flexibles formant la branche inspiratoire et la branche expiratoire, par exemple des tuyaux en polymère.
- la branche inspiratoire et la branche expiratoire, e.g. des conduites flexibles, sont raccordées à une pièce de jonction, telle une pièce en Y.
- la branche inspiratoire et/ou la branche expiratoire sont reliées fluidiquement à une interface respiratoire patient, de préférence via la pièce de jonction.
- l'interface respiratoire patient comprend une sonde d'intubation trachéale ou masque respiratoire, ou autre.
- la branche inspiratoire et la branche expiratoire sont en outre fluidiquement raccordées à, respectivement, des orifices de sortie et d'entrée du ventilateur médical.
- le circuit respiratoire, en particulier la branche inspiratoire, peut comprendre un humidificateur de gaz.
- l'humidificateur de gaz est agencé en aval du dispositif d'injection, de manière à pouvoir humidifier le gaz avant son administration par inhalation au patient.
- le ventilateur médical est alimenté électriquement par une ou des sources de courant électrique, typiquement le secteur (110/220V) et/ou une ou des batteries rechargeables.
- le ou les récipients de NO, i.e. la ou les bouteilles de NO, et/ou l'appareil de délivrance de NO sont agencés sur un chariot de transport à roulettes.

Selon un autre aspect, l'invention concerne aussi une méthode de traitement thérapeutique d'une personne, i.e. un patient humain (i.e. adulte, enfant, adolescent ou nouveau-né), souffrant d'hypertension pulmonaire et/ou d'hypoxie, engendrant des vasoconstrictions pulmonaires ou analogues, comprenant une administration par inhalation à la personne en ayant besoin, d'un mélange gazeux comprenant de 1 à 80 ppmv de NO et au moins 20 %vol. d'oxygène environ, de préférence au moins 21 %vol. d'oxygène environ, au moyen d'une installation de fourniture de gaz, telle celle décrite ci-avant selon l'invention, comprenant un appareil de délivrance de NO assurant une délivrance de NO à la posologie souhaitée, de manière à traiter (au moins partiellement) ladite hypertension pulmonaire et/ou ladite hypoxie, qui peut être causée par une (ou des) pathologie ou autres troubles pulmonaires typiquement de type PPHN (hypertension pulmonaire persistante du nouveau-né) ou SDRA (syndrome de détresse respiratoire aigüe), ou encore engendrée par une opération de chirurgie cardiaque avec mise du patient sous circulation sanguine extracorporelle (CEC).

### Définitions

D'une façon générale, dans le cadre de l'invention :
- « ppmv » signifie partie par million en volume,
- « %vol. » » signifie pourcentage en volume.
- « NO » désigne le monoxyde d'azote.
- « NO₂ » désigne le dioxyde d'azote.
- « N₂ » désigne l'azote.
- « O₂ » désigne l'oxygène.
- les pressions sont exprimées en bars absolus, abrévié « bars ».
- les termes « concentration », « quantité », « proportion », « dose » et « teneur » sont considérés comme équivalents et substituables.
- les termes « conduit », « tuyau » ou « ligne » sont considérés comme équivalents et substituables.
- les termes « moyen de/à/pour » sont considérés comme totalement équivalents et substituables par les termes « dispositif de/à/pour », par exemple les termes « moyens de pilotage » peuvent être remplacés par « dispositif de pilotage », les termes « moyens à vannes » peuvent être remplacés par « dispositif à vannes », les « moyens de mémorisation» peuvent être remplacés par « dispositif de mémorisation» ...
- par « mesure de pression », on entend une valeur de pression (e.g. une valeur numérique) ou un signal représentatif d'une telle valeur de pression reflétant ou correspondant à la pression gazeuse mesurée par un capteur de pression ou analogue.
- les termes « amont » et « aval » sont utilisés par rapport au sens normal de circulation du gaz, à savoir dans le sens allant des récipients de gaz vers l'appareil de délivrance, puis vers le patient.
- un récipient de NO, telle une bouteille de gaz, est dit « vide » ou « quasiment vide » lorsqu'il contient une faible quantité ou pression de gaz à base de NO car l'essentiel du gaz qu'il contenait a été consommé/utilisé, par exemple si le récipient contient une quantité de gaz correspondant à une pression gazeuse préfixée, par exemple inférieure ou égale à 25 bars de gaz à base de NO.

L'invention va maintenant être mieux comprise grâce à la description détaillée suivante, faite à titre illustratif mais non limitatif, en référence aux figures annexées parmi lesquelles :
Figure 1 schématise un mode de réalisation d'une installation d'administration de gaz selon l'invention.
Figure 2 schématise un mode de réalisation d'une partie de l'architecture interne de l'appareil de délivrance de NO d'une installation d'administration de gaz selon l'invention, telle celle de Figure 1.

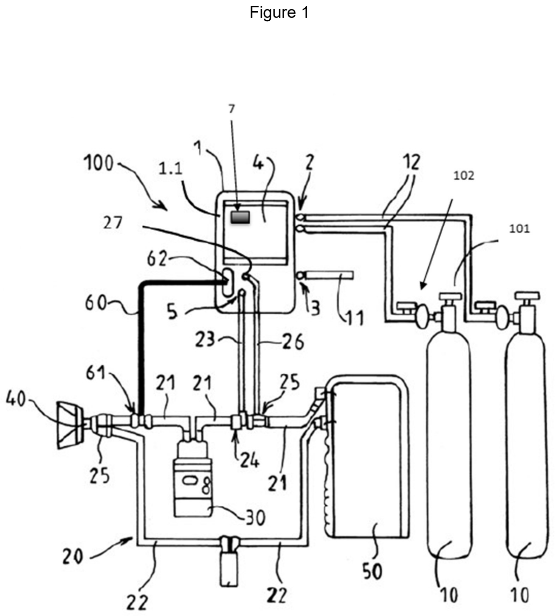

La Figure 1 schématise un mode de réalisation d'une installation d'administration de gaz 100 selon l'invention comprenant un appareil de fourniture de NO 1 fournissant un mélange gazeux à base de monoxyde d'azote (NO), et un ventilateur médical 50 fournissant un gaz contenant au moins 20%vol. d'oxygène, tel de l'air ou autre.

L'installation 100 comprend des sources de NO, à savoir ici deux récipients ou bouteilles de gaz sous pression 10 contenant chacune un gaz à base de NO, c'est-à-dire un mélange gazeux NO/N₂, à savoir ici un mélange gazeux NO/N₂ contenant entre 100 et 5000 ppmv de NO (reste N₂), de préférence entre 100 et 1000 ppmv de NO (reste N₂), par exemple 450 ou 800 ppm vol. de NO (reste N₂), ou toute autre concentration adéquate, qui alimentent en mélange NO/N₂, le dispositif ou appareil 1 de délivrance ou fourniture de NO permettant de suivre et contrôler la fourniture du mélange gazeux NO/N₂.

Le mélange gazeux NO/N₂ est généralement conditionné dans chaque récipients 10 à une pression, appelée « pression haute », d'au moins 150 bars, laquelle correspond à la pression de départ régnant dans chaque récipient avant le début de tout soutirage, c'est-à-dire mesurée avant tout soutirage ou toute utilisation de gaz (i.e. récipient plein). Dans certains cas, elle peut atteindre 230 à 250 bars, voire même plus. Bien entendu, la pression du gaz dans les récipients 10 diminue au fur et à mesure du soutirage du gaz, c'est-à-dire de l'utilisation progressive du gaz, engendrant ainsi une vidange progressive des récipients 10.

Comme illustré en Figure 1, chaque récipient 10 comprend un corps de forme générale cylindrique ou d'ogive, contenant le gaz au sein de son volume interne, lequel corps est surmonté d'un robinet 101 de distribution du gaz servant à contrôler la sortie du gaz du volume interne du corps du récipient 10.

Chaque robinet 101 est un robinet dit « simple », c'est-à-dire ne contenant pas de moyens de détente interne. De ce fait, des moyens de réduction de pression externes, c'est-à-dire un dispositif de détente 102, aussi appelé détendeur de gaz, sont agencés en aval de chaque robinet 101 afin d'assurer une réduction de la pression gazeuse, à une pression de détente (PD) souhaitée qui est réglée au niveau de chaque détendeur, typiquement une pression de détente inférieure ou égale à 10 bars, de préférence inférieure ou égale à 8 bars, typiquement entre 4 et 7 bars, avantageusement entre 5 et 6 bars. Chaque dispositif de détente 102 est par exemple vissé sur la sortie en pression du robinet 101 sur lequel il est agencé, c'est-à-dire qu'il est monté sur le raccord de sortie en pression du détendeur 102.

Chaque bouteille de gaz 10 est reliée fluidiquement à l'appareil 1 de fourniture de NO, via un conduit flexible 12, c'est-à-dire une ligne d'amenée de gaz, typiquement des tuyaux flexibles ou analogues, qui peuvent être équipées de dispositifs de suivi de la pression de gaz, notamment un ou des manomètres ou autres. L'extrémité amont du conduit flexible 12 vient se raccorder, comme illustré en Figure 1, à la sortie du dispositif de détente 102.

Alternativement, selon un autre mode de réalisation (non montré), les robinets 101 pourraient être du type à détendeur intégré (RDI), c'est-à-dire que les moyens de détente 102 seraient alors agencés dans (i.e. intégrés) le corps de chaque robinet 101, par exemple un clapet de détente coopérant avec un siège de clapet. Dans ce cas, le gaz sortant du robinet 101 est à la pression de détente souhaitée, i.e. à une pression de détente inférieure ou égale à 10 bars, comme précédemment, et le (les) conduit flexible 12 vient se raccorder directement à la sortie en pression dudit robinet 101.

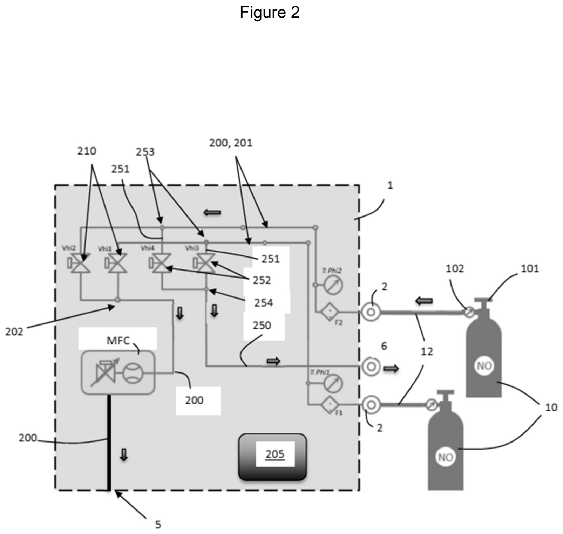

Chaque conduit flexible 12 achemine le gaz à base de NO à la pression de détente (PD), c'est-à-dire le gaz détendu, jusqu'à l'appareil 1. Il vient se raccorder fluidiquement à une entrée de gaz de l'appareil 1, appelée entrée de NO 2, qui alimente le circuit de gaz principal 200 interne de l'appareil 1, comme détaillé en Figure 2, servant à acheminer le gaz à base de NO au sein de l'appareil de fourniture de NO 1, c'est-à-dire au sein de la coque ou carcasse 1.1 périphérique de l'appareil 1, entre chaque entrée de NO 2 et une (ou plusieurs) sortie de NO 5.

Chaque entrée de NO 2 est configurée pour permettre d'y raccorder fluidiquement l'extrémité aval d'un conduit flexible 12. Pour ce faire, on peut utiliser des moyens de connexion fluidique classiques, comme un raccord fluidique, un connecteur fluidique ou analogue, auxquels vient se fixer, par exemple par emboitement, le conduit flexible 12.

Dans le mode de réalisation de Figure 2, le circuit principal de gaz 200 comprend deux tronçons d'entrée 201, agencés en parallèle l'un de l'autre, et deux entrées de NO 2, chaque tronçon d'entrée 201 étant relié fluidiquement à l'une des entrées de NO 2 alimentée en mélange NO/N₂ par un conduit flexible 12 amenant le gaz (i.e. NO/N₂) depuis l'un ou l'autre des récipients de gaz 10. Les deux tronçons d'entrée 201 du circuit principal de gaz 200 viennent se raccorder fluidiquement l'un à l'autre en un site de jonction 202.

Le circuit principal de gaz 200, en particulier chaque tronçon d'entrée 201, est équipé de premiers moyens de contrôle de flux 210, typiquement des vannes de contrôle Vhi1, Vhi2 qui sont pilotées par des moyens de pilotage de l'appareil 1 de manière à contrôler le flux de gaz au sein du circuit principal de gaz 200, en particulier de chaque tronçon d'entrée 201. Les moyens de pilotage 205 agissent donc sur les moyens de contrôle de flux 210, typiquement des vannes de contrôle Vhi1, Vhi2.

Les premiers moyens de contrôle de flux 210 sont donc agencés entre chaque entrée de NO 2 et le site de jonction 202 des deux tronçons 201. Préférentiellement, les vannes de contrôle pilotées Vhi1, Vhi2 sont des électrovannes, appelées électrovannes principales Vhi1, Vhi2, ou des vannes pilotées analogues.

Plus précisément, les moyens de pilotage 205 peuvent piloter les vannes de contrôle Vhi1, Vhi2, c'est-à-dire les électrovannes principales, pour les passer être en position ouverte et ainsi laisser passer du gaz au travers de l'une ou l'autre des vannes ou, à l'inverse, en position fermée et ainsi interdire tout passage de gaz au travers de l'autre des vannes, et inversement. Les moyens de pilotage peuvent piloter les vannes de contrôle Vhi1, Vhi2 pour qu'elles soient toutes les deux en position fermée afin d'empêcher, i.e. interdire ou stopper, toute circulation du gaz dans le reste du circuit principal 200, c'est-à-dire notamment en aval du site de jonction 202.

D'une façon générale, lorsque l'une ou l'autre des vannes de contrôle Vhi1, Vhi2 est en position ouverte, la circulation du gaz à base de NO dans le circuit principal de gaz 200 se fait dans le sens allant des entrées de NO 2 vers les premiers moyens de contrôle de flux 210, i.e. les vannes de contrôle Vhi1, Vhi2.

De manière habituelle, les moyens de pilotage 205 de l'appareil 1 commandant les vannes de contrôle Vhi1, Vhi2 pilotées comprennent un (ou des) microprocesseurs mettant en oeuvre un (ou des) algorithmes ou autres programmes informatiques. Les microprocesseurs sont portés par une carte électronique. La carte électronique peut aussi comprendre des moyens de mémorisation, telle une mémoire flash ou analogue.

Les moyens de pilotage 205 sont reliés électriquement, via des liaisons électriques ou analogues, tels des câbles électriques, aux premiers moyens de contrôle de flux 210, typiquement aux vannes de contrôle Vhi1, Vhi2 pilotées.

La pression gazeuse qui règne dans le circuit 200, en particulier dans les deux tronçons d'entrée 201 correspond à la pression gazeuse acheminée par les conduits flexibles 12, donc à la pression de détente. Cette pression du gaz au sein au sein des deux tronçons d'entrée 201 est suivie en permanence, i.e. mesurée par, des moyens de mesure de pression Phi1, Phi2, tels des capteurs de pression, agencés sur ces tronçons 201, permettant d'opérer des mesures de pression au sein des tronçons 201 du circuit 200, et fournir ces mesures aux moyens de pilotage qui les traitent.

Avantageusement, le flux gazeux entrant dans le circuit 200, en particulier dans les deux tronçons d'entrée 201, peut être filtré par des moyens de filtration F1, F2, tels des filtres HEPA ou autres, agencés dans les deux tronçons d'entrée 201, à proximité des entrées de NO 2.

Par ailleurs, l'appareil de délivrance de NO 1 de l'installation 100 comprend aussi une entrée d'oxygène 3 reliée fluidiquement, via un conduit d'amenée d'oxygène 11, tel un tuyau flexible ou analogue, à une source d'oxygène (non montrée), par exemple une bouteille d'oxygène sous pression ou un réseau hospitalier, c'est-à-dire une canalisation d'alimentation en oxygène agencée dans un bâtiment hospitalier. Ceci permet d'alimenter le circuit de gaz interne 200 en oxygène quand cela est nécessaire.

L'installation 100 d'administration de gaz de Figure 1 comprend en outre un ventilateur médical 50, c'est-à-dire un appareil d'assistance respiratoire, fournissant un flux de gaz respiratoire à base d'oxygène, c'est-à-dire contenant au moins 20%vol. d'oxygène environ, préférentiellement au moins 21%vol. d'oxygène environ, tel de l'air ou un mélange oxygène/azote (N₂/O₂).

Le ventilateur médical 50 et l'appareil de délivrance de NO 1 de l'installation 100 sont en communication fluidique avec un circuit respiratoire 20, aussi appelé circuit patient, en particulier avec la branche inspiratoire 21 du circuit 20, qui sert à acheminer le flux gazeux vers l'interface respiratoire 40 fournissant le flux gazeux thérapeutique au patient, c'est-à-dire un mélange gazeux final ou combiné contenant la posologie en NO souhaitée. Le mélange gazeux combiné à administrer au patient est formé par mélange du flux gazeux à base d'oxygène (e.g. air ou mélange O₂/N₂) provenant du ventilateur médical 50 et du flux contenant le NO, typiquement un mélange gazeux NO/N₂, délivré par l'appareil de délivrance de NO 1.

Pour ce faire, l'appareil de délivrance de NO 1 fournit ou injecte le mélange NO/N₂ dans la branche inspiratoire 21 du circuit 20, via un conduit d'injection 23, reliant fluidiquement le circuit principal de gaz 200 interne de l'appareil de délivrance de NO 1 à un dispositif d'injection 24 agencé sur la branche inspiratoire 21.

Le dispositif d'injection 24 est configuré pour opérer un mélange du gaz contenant du NO provenant de l'appareil de délivrance de NO 1 avec le flux de gaz respiratoire provenant du ventilateur 50 et obtenir un mélange gazeux combiné contenant du NO et de l'oxygène, lequel est ensuite administré au patient.

Le mélange gazeux combiné contient du NO à la posologie désirée, de l'azote (N₂) et de l'oxygène (O₂), c'est-à-dire un mélange gazeux NO/N₂/O₂. Eventuellement, peuvent être présentes des impuretés inévitables, e.g. argon, CO₂, NO₂...

La branche inspiratoire 21 du circuit 20 comprend en outre un humidificateur de gaz 30 agencé en aval du dispositif d'injection 24 servant à humidifier le flux de gaz combiné NO/N₂/O₂, avant qu'il ne soit administré par inhalation au patient à traiter, via l'interface respiratoire 40, telle une sonde d'intubation trachéale, un masque respiratoire ou analogue.

Le circuit 20 comprend une branche expiratoire 22 reliée fluidiquement à la branche inspiratoire 21 et à l'interface respiratoire 40, via une pièce de jonction 25, typiquement une pièce en Y.

Par ailleurs, un capteur de débit 25, par exemple du type à fil chaud, à différentiel de pression ou massique, est agencé sur la branche inspiratoire 21, entre le ventilateur 50 et le dispositif d'injection 24. Le capteur de débit 25 est relié à un port de connexion 27 de l'appareil de délivrance de NO 1, via une (des) ligne de mesure de débit 26 venant se raccorder audit port de connexion au capteur 27, c'est-à-dire un ou des câbles électriques ou analogues. Le capteur de débit 25 sert à mesurer le débit de gaz délivré par le ventilateur 50, tel de l'air ou N₂/O₂, circulant dans la branche inspiratoire 21, en amont du dispositif d'injection. Ces mesures de débit opérées par le capteur de débit 25 permettent de contrôler ou réguler plus efficacement la délivrance du flux de NO (i.e. N₂/O₂) par l'appareil de délivrance de NO 1, en particulier le débit de NO. Les mesures de débit opérées par le capteur de débit 25 sont traitées par les moyens de pilotage 205 à (micro)processeur de l'appareil de délivrance de NO 1, typiquement un (micro)contrôleur.

Comme schématisé en Figure 2, en fonctionnement normal, le flux de NO/N₂ chemine par le circuit principal de gaz 200 de l'appareil 1, notamment via un contrôleur de débit massique ou MFC (Mass Flow Controller) agencé en aval du site de jonction 202, avant de sortir du circuit principal de gaz 200 par la sortie de NO 5 de l'appareil 1. De préférence, le MFC comprend une électrovanne proportionnelle et un capteur de débit additionnel coopérant avec les moyens de pilotage 205.

Les moyens de pilotage 205 permettent notamment d'ajuster ou contrôler le débit de gaz à base de NO en pilotant le MFC, en particulier l'électrovanne proportionnelle. En particulier, en fonctionnement, ils peuvent déterminer le débit de NO à fournir pour obtenir la teneur en NO souhaitée dans le mélange combiné, c'est-à-dire la posologie en NO désirée, à partir notamment de la consigne de teneur en NO réglée et/ou fixée par l'utilisateur, de la composition du mélange gazeux NO/N₂ provenant des bouteilles de gaz 10, et d'une (ou des) mesure de débit opérée(s) par le capteur de débit 25 agencé sur la branche inspiratoire 21.

D'une façon générale, les moyens de pilotage 205 permettent en outre de traiter des mesures, d'opérer des calculs et/ou de contrôler ou commander tous les éléments électromécaniques de l'appareil 1, tels que les électrovannes, les affichages ...

Le circuit de gaz interne 200 de l'appareil de délivrance de NO 1 peut aussi comprendre d'autres éléments ou composants, en particulier un (ou des) capteur de pression, un (ou des) capteur de débit additionnel ou débitmètre et/ou des dispositifs à orifice calibré ou autres, et préférentiellement une ligne de bipasse ou de secours permettant de bipasser le MFC lorsqu'il présente un dysfonctionnement par exemple ou en cas d'interruption de signal provenant du capteur de débit 25 agencé sur la branche inspiratoire 21.

L'appareil de fourniture de NO 1 comprend aussi une interface graphique utilisateur ou IGU comprenant un afficheur graphique 4, de préférence un écran tactile, c'est-à-dire à dalle tactile, servant à afficher différentes informations ou données, icones, courbes, alarmes..., ainsi que des touches de sélection virtuelles et/ou des pavés ou fenêtres, servant notamment à opérer des choix, des sélections , des validations ou annulations de choix ou encore à entrer des informations, telles des valeurs désirées (e.g. débit, dosage de NO...), ou toute autre information ou donnée utile au personnel soignant.

De préférence, l'affichage est en couleurs mais il peut se faire aussi en noir et blanc.

L'alimentation électrique de l'appareil de fourniture de NO 1, en particulier des composants nécessitant du courant électrique pour fonctionner, tels les moyens de pilotage 205, l'afficheur graphique 4..., est assurée classiquement par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant. L'alimentation électrique du ventilateur médical 50 est assurée de façon analogue, notamment par une liaison au courant du secteur ou une batterie interne.

En outre, l'installation 100 comprend aussi une ligne de prélèvement de gaz 60 qui relie fluidiquement la branche inspiratoire 21 (en 61) à un port d'entrée 62 du dispositif de fourniture de NO 1, tel un tuyau flexible ou analogue. Elle permet de prélever des échantillons de gaz et de les convoyer jusqu'au dispositif de fourniture de NO 1 où ils sont analysés dans un analyseur de gaz interne (non montré), c'est-à-dire au sein d'une ligne de calibration comprenant des capteurs de NO₂, de NO et d'O₂, notamment des cellules électrochimiques, reliés électriquement aux moyens de pilotage 205 afin de vérifier que la composition du gaz final est conforme à celle du mélange gazeux NO/N₂/O₂ souhaité, notamment qu'il ne contient pas de quantité excessive d'espèces NO₂ toxiques, que sa teneur en oxygène n'est pas hypoxique et que sa teneur en NO correspond à la posologie souhaitée, i.e. dose de NO à administrer. Les moyens de pilotage 205 récupèrent et traitent les signaux des capteurs et peuvent d'agir en réponse à ces signaux, notamment pour opérer une calibration des capteurs ou déclencher une alarme en cas de teneur non-conforme.

Au fur et à mesure du traitement du patient, chaque récipient de NO 10, i.e. bouteille de NO, se vide et la pression qui y règne diminue donc progressivement jusqu'à ce que le récipient soit vide ou quasiment vide, c'est-à-dire lorsqu'il contient une faible quantité de gaz à base de NO car l'essentiel du gaz qu'il contenait a été consommé/utilisé pendant le traitement du patient, typiquement, lorsque le récipient de NO contient une quantité de gaz correspondant à une pression gazeuse préfixée, dite pression « résiduelle », par exemple moins de 25 bars de gaz à base de NO résiduel. Bien entendu, selon le mode de réalisation, la pression gazeuse « résiduelle » préfixée, i.e. le seuil de pression résiduelle, peut une autre valeur, c'est-à-dire être supérieure à 25 bars ou inférieure à 25 bars.

Dans le mode de réalisation proposé sur les Figures, on considère que la pression gazeuse préfixée dite « résiduelle » est égale à 25 bars environ.

Comme déjà expliqué, pour remplacer une bouteille de NO 10 vide par une nouvelle bouteille de NO 10 pleine, c'est-à-dire une bouteille « neuve », il faut pouvoir déconnecter la bouteille de NO 10 qui est vide du conduit flexible 12 qui la relie fluidiquement à l'appareil de délivrance de NO 1 et plus précisément de déconnecter le conduit flexible 12 du détendeur de gaz 102, fixé au robinet 101 de la bouteille 10 vide à changer, auquel vient se fixer le conduit flexible 12.

Or, cela n'est possible tant que l'utilisateur n'a pas purgé la pression résiduelle, de bars, par exemple de 2 à 7 bars, qui règne dans le tuyau flexible 12 entre l'appareil 1 et le détendeur 102 de la bouteille de NO 10 à remplacer.

Pour ce faire, afin d'éviter les problèmes et inconvénients rencontrés avec les installations de l'art antérieur, selon la présente invention, l'appareil de délivrance de NO est équipé en outre d'un circuit de purge 250 venant se raccorder fluidiquement (en 253) au circuit de gaz principal 200, en particulier aux deux tronçons 201, entre la ou les entrées de NO 2 et les premiers moyens de contrôle de flux 210.

Le circuit de purge 250 comprenant deux tronçons de jonction 251 raccordés fluidiquement (en 253) aux deux tronçons d'entrée 201 au circuit de gaz principal 200, et par ailleurs une sortie de purge 6 en communication fluidique avec l'atmosphère ambiante, c'est-à-dire avec l'air de la pièce.

Les deux tronçons de jonction 251 se réunissent en un site de réunion 254 situé sur le circuit de purge 250, en amont de la sortie de purge 6.

Par ailleurs, des seconds moyens de contrôle de flux 252 sont agencés sur ledit circuit de purge 250, en particulier sur les deux tronçons de jonction 251 du circuit de purge 250, en amont du site de réunion 254. Ces seconds moyens de contrôle de flux 252 permettent de contrôler la circulation de gaz dans ledit circuit de purge 250 en direction de la sortie de purge 6, typiquement dans les deux tronçons de jonction 251 qui sont agencés en parallèle l'un de l'autre.

Comme précédemment, les seconds moyens de contrôle de flux 252 comprennent typiquement des vannes de contrôle Vhi3, Vhi4 qui sont pilotées par les moyens de pilotage 205 de l'appareil 1 de manière à contrôler le flux de gaz au sein du circuit de purge 250, en particulier dans chaque tronçon d'entrée 201. Les moyens de pilotage 205 agissent donc sur les seconds moyens de contrôle de flux 252, c'est-à-dire sur les vannes de contrôle Vhi3, Vhi4, pour autoriser ou interdire/stopper la circulation de gaz. Là encore, les vannes de contrôle Vhi3, Vhi4 sont préférentiellement des électrovannes, appelées électrovannes de purge, ou des vannes pilotées analogues, et sont pilotées par les moyens de pilotage 205 pour les passer être en position ouverte et ainsi laisser passer du gaz au travers de l'une ou l'autre des vannes ou, à l'inverse, en position fermée et ainsi interdire tout passage de gaz au travers de l'autre des vannes, et inversement. Les moyens de pilotage 205 peuvent piloter les vannes de contrôle Vhi3, Vhi4, c'est-à-dire les électrovannes de purge, pour qu'elles soient toutes les deux en position fermée afin d'empêcher, i.e. interdire ou stopper, toute circulation du gaz dans le reste du circuit de purge 250, c'est-à-dire en direction de la sortie de purge 6.

En outre, l'appareil 1 comprend également des moyens de déclenchement de purge 7, actionnables, i.e. activables, par l'utilisateur, permettant de débuter une procédure de purge, c'est-à-dire d'indiquer aux moyens de pilotage 205 qu'il faut contrôler les premiers et seconds moyens de contrôle de flux 210, 252, typiquement les électrovannes principales Vhi1, Vhi2 et secondaires Vhi3, Vhi4, afin de contrôler les flux gazeux circulant dans le circuit principal 200 et le circuit de purge 250.

Avantageusement, les moyens de déclenchement de purge 7 comprennent une touche de lancement de purge 7.1 virtuelle, i.e. digitale, s'affichant sur l'écran d'affichage tactile 4 de l'appareil 1. Cette touche de lancement de purge 7.1 virtuelle peut être « activée » par un appui ou contact du doigt par l'utilisateur, par exemple de l'index.

Lorsqu'elle est « activée », le contact du doigt sur la zone concernée de l'écran tactile 4 est détecté et un signal d'activation est transmis aux moyens de pilotage 205 qui l'interprètent alors comme un signal de début de purge, i.e. de lancement de purge de conduit flexible en vue du remplacement d'une des bouteilles de gaz 10.

En réponse à un appui de l'utilisateur sur la touche de lancement de purge virtuelle 7.1 et au signal de début de purge, les moyens de pilotage 205 sont configurés pour piloter les électrovannes principales et de purge Vhi1, Vhi2 ; Vhi3, Vhi4, de manière :
- à stopper la circulation de gaz dans la partie du circuit de gaz principal 200 situées en aval des électrovannes principales Vhi1, Vhi2, c'est-à-dire dans une partie des tronçons d'entrée 201, en fermant ou maintenant en position de fermeture, les deux électrovannes principales Vhi1, Vhi2.
- à maintenir en position de fermeture l'une des électrovannes de purge (Vhi3, Vhi4) et
- et à permettre une circulation de gaz dans le circuit de purge (250) en passant l'autre des électrovannes de purge (Vhi3, Vhi4) en position d'ouverture de manière à mettre le tronçon de purge 251 correspondant, le tronçon d'entrée 201 qui lui est relié fluidiquement et le conduit flexible 12 qui lui est raccordé à ce dernier, en communication fluidique les uns avec les autres, et par ailleurs avec l'atmosphère, via l'orifice de purge 6, de manière purger la pression gazeuse présente dans le conduit flexible 12 et dans le tronçon d'entrée 201 du circuit principal 200, et ainsi permettre à l'utilisateur de démonter ensuite sans risque le conduit flexible 12 du dispositif détendeur 102 et ce dernier de la bouteille 10 à remplacer puisqu'elle est vide ou quasiment vide.

Selon un mode de réalisation particulier, les moyens de pilotage 205 sont configurés pour piloter les électrovannes principales et de purge Vhi1, Vhi2 ; Vhi3, Vhi4 et démarrer une purge après un premier appui sur la touche de lancement de purge 7.1, suivi d'un second appui sur une touche de validation virtuelle (non montrée) s'affichant sur l'écran 4, i.e. une touche de confirmation de choix.

Ces touches de lancement de purge et de validation virtuelle peuvent s'afficher au sein d'une fenêtre s'affichant sur l'écran 4 de l'appareil.

Selon un mode de réalisation, les bouteilles de NO 10 et/ou l'appareil de délivrance de NO 1 peuvent être agencés sur un chariot de transport (non montré), tel celui décrit par EP4159585 ou EP4461619.

D'une façon générale, i.e. non limitée aux mode de réalisation des Figures, l'invention porte aussi sur une méthode pour remplacer un récipient de NO (quasiment) vide, appelé ci-après bouteille de NO (quasiment) vide 10, raccordé fluidiquement par un tuyau ou conduit flexible 12 au circuit principal 200 de gaz de l'appareil de délivrance de NO 1 d'une installation d'administration de gaz 100 selon l'invention, telle celle de la Figure 1, par une bouteille de NO pleine (i.e. récipient « neuf »), ledit conduit flexible 12 contenant du NO gazeux, typiquement un mélange NO/N₂, à une pression résiduelle supérieure 1 bar, dans laquelle :
a) on débute une procédure de purge dudit conduit flexible 12 par appui digital (i.e. du doigt) d'un utilisateur sur une touche de lancement de purge 7.1 virtuelle affichée sur l'écran d'affichage tactile 4 de l'appareil 1 de délivrance de NO 1, de manière à générer (i.e. du fait de l'appui digital) un signal d'activation transmis aux moyens de pilotage 205 de l'appareil 1 de délivrance de NO 1,
b) en réponse audit signal d'activation, les moyens de pilotage 205 de l'appareil de délivrance de NO 1 contrôlent les flux gazeux, i.e. NO gazeux, circulant dans le circuit principal 200 et le circuit de purge 250 de l'appareil de délivrance de NO 1, de manière à :
   - stopper la circulation de gaz dans la partie du circuit de gaz principal 200 située en aval d'au moins une électrovanne principale Vhi1, Vhi2 agencée sur ledit circuit de gaz principal 200 de l'appareil de délivrance de NO 1, en fermant ou maintenant en position de fermeture ladite au moins une électrovanne principale Vhi1, Vhi2, et
   - autoriser une circulation de gaz dans le circuit de purge 250 de l'appareil de délivrance de NO 1 en passant une (première) électrovanne de purge Vhi3, Vhi4 en position d'ouverture de manière à mettre la portion de circuit de gaz principal 200 reliée fluidiquement au conduit flexible 12 à purger, en communication fluidique avec l'atmosphère, i.e. via un orifice de purge 6, de manière évacuer vers l'atmosphère, au moins une partie du NO gazeux à la pression résiduelle présent dans le conduit flexible 12 à purger et dans ladite portion de circuit principal 200,
c) opérer une déconnection (i.e. démontage) par l'utilisateur du conduit flexible 12 de la bouteille vide 10 à remplacer et/ou d'un dispositif détendeur 102 agencé entre le conduit flexible 12 et la bouteille vide 10 à remplacer, et
d) opérer une (re)connexion (i.e. remontage) par l'utilisateur du conduit flexible 12 à une bouteille pleine 10, i.e. récipient 'neuf' de remplacement, et/ou au dispositif détendeur 102 agencé entre le conduit flexible 12 et la bouteille pleine 10.

Aux étapes c) et d), selon le mode de réalisation, le conduit flexible 12 peut être relié/raccordé fluidiquement :
- directement à la bouteille de NO 10, typiquement à un robinet (aussi appelé vanne ou analogue) équipant la bouteille de gaz et incluant des moyens de détente de gaz interne, à savoir un robinet à détendeur intégré ou RDI, permettant de réduire la pression du gaz et de le fournir à une pression souhaitée, par exemple moins de 10 bar (non illustré) ; ou
- indirectement à la bouteille de NO 10, en étant raccordé à un dispositif détendeur 102 externe lui-même agencé entre le conduit flexible 12 et la bouteille de gaz 10, typiquement fixé au robinet équipant la bouteille de gaz, comme illustré en Fig. 1. Dans ce cas, c'est le dispositif détendeur 102 qui assure une réduction de la pression du gaz et sa fourniture à la pression souhaitée, e.g. moins de 10 bar.

Selon le mode de réalisation considéré, la méthode de l'invention peut comprendre l'une ou plusieurs des caractéristiques suivantes :
- elle comprend en outre un maintien en position de fermeture d'une seconde électrovanne de purge Vhi3 faisant partie des électrovannes de purge Vhi3, Vhi4, lorsque la première électrovanne de purge Vhi4, i.e. l'autre électrovanne de purge, est en position d'ouverture.
- à l'étape a), l'appui digital (i.e. du doigt) de l'utilisateur sur la touche de lancement de purge 7.1 virtuelle doit être suivi d'un second appui sur une touche de validation virtuelle (i.e. de confirmation de choix) s'affichant sur l'écran 4 pour générer le signal d'activation.
- lors du remplacement d'une bouteille vide par une bouteille pleine, pour éviter toute interruption de traitement du patient, une autre bouteille de NO pleine prend le relais pour fournir le mélange NO/N₂ à l'appareil de délivrance de NO, en particulier à une partie du circuit principal 200 qui n'est pas en communication fluidique avec la portion de circuit de gaz principal reliée fluidiquement à l'atmosphère via le orifice de purge 6.
- les électrovannes principales Vhi1, Vhi2 et secondaires Vhi3, Vhi4 font partie des moyens de contrôle de flux 210, 252 de l'appareil de délivrance de NO 1.
- en réponse à l'appui de l'utilisateur sur la touche de lancement de purge virtuelle 7.1 et au signal de début de purge, les moyens de pilotage 205 sont configurés pour piloter les électrovannes principales et de purge Vhi1, Vhi2 ; Vhi3, Vhi4.
- un tuyau flexible 12 au circuit principal 200 de gaz de l'appareil de délivrance de NO 1.
- deux bouteilles de NO 10 sont raccordées fluidiquement chacune par un tuyau flexible 12 au circuit principal 200 de gaz de l'appareil de délivrance de NO 1.
- lorsque l'une des bouteilles de NO 1 est vide et en cours de remplacement, l'autre alimente le circuit principal 200 de gaz de l'appareil de délivrance de NO 1 avec un flux de NO gazeux, i.e. de mélange NO/N₂.
- la ou les bouteilles de NO 10 contiennent un mélange gazeux NO/N₂.
- la ou les bouteilles de NO 10 sont équipées d'un robinet de distribution de gaz 101.
- un dispositif détendeur de gaz est fixé sur le robinet de distribution de gaz 101 de la ou les bouteilles de NO 10.
- chaque tuyau flexible est raccordé fluidiquement à un dispositif détendeur de gaz.
- le remplacement d'une bouteille vide par une bouteille pleine, comprend les opérations de :
   ▪ débrancher le conduit ou tuyau flexible du dispositif détendeur,
   ▪ de démonter, notamment de dévisser, le dispositif détendeur de la bouteille vide, typiquement du robinet équipant la bouteille vide,
   ▪ de remonter, notamment de visser, le dispositif détendeur sur la bouteille de NO pleine de remplacement, typiquement sur le robinet équipant la bouteille pleine, et
   ▪ de (re)connecter fluidiquement le conduit ou tuyau flexible au dispositif détendeur.
- les bouteilles de NO 10 et/ou l'appareil de délivrance de NO 1 sont agencés sur un chariot de transport.

D'une façon générale, une installation d'administration de gaz 100 selon l'invention peut être utilisée pour administrer par inhalation du monoxyde d'azote (NO), i.e. le mélange final obtenu NO/O₂/N₂, aux personnes, i.e. patients, souffrant d'hypertension artérielle pulmonaire aiguë, notamment pour opérer une dilatation de leurs vaisseaux pulmonaires et une augmentation de leur oxygénation en améliorant les échanges gazeux pulmonaires, en particulier pour traiter l'Hypertension Artérielle Pulmonaire du Nouveau-né ou PPHN, le Syndrome de Détresse Respiratoire Aigüe ou SDRA observé principalement chez l'adulte, ou les hypertensions pulmonaires (HP) en chirurgie cardiaque chez l'adulte ou l'enfant.

## Revendications

1. Appareil de délivrance de NO (1) pour fournir un gaz contenant du NO comprenant :
- un circuit de gaz principal (200) pour véhiculer le gaz contenant du NO entre au moins une entrée de NO (2) et au moins une sortie de NO (5),
- ladite au moins une entrée (2) de NO configurée pour permettre d'y raccorder fluidiquement un conduit flexible (12) de manière à alimenter le circuit de gaz principal (200) avec le gaz contenant du NO amené par ledit conduit flexible (12),
- des premiers moyens de contrôle de flux (210) agencés sur ledit circuit de gaz principal (200), configurés pour contrôler la circulation du flux de gaz contenant du NO dans ledit circuit de gaz principal (200),
- des moyens de pilotage (205) à microprocesseur,
- un circuit de purge (250) raccordé fluidiquement (en 253) au circuit de gaz principal (200) entre ladite au moins une entrée de NO (2) et les premiers moyens de contrôle de flux (210), ledit circuit de purge (250) comprenant une sortie de purge (6) en communication fluidique avec l'atmosphère, et
- des seconds moyens de contrôle de flux (252) agencés sur ledit circuit de purge (250), configurés pour contrôler la circulation de gaz dans ledit circuit de purge (250) en direction de la sortie de purge (6), et
- des moyens de déclenchement de purge (7), actionnables par l'utilisateur,
**caractérisé en ce que** :
- il comprend en outre un écran d'affichage tactile (4),
- les moyens de déclenchement de purge (7) comprennent une touche de lancement de purge virtuelle (7.1) s'affichant sur l'écran d'affichage tactile (4) et
- les moyens de pilotage (205) sont configurés pour piloter les premiers et seconds moyens de contrôle de flux (210 ; 252) de manière à stopper la circulation de gaz dans au moins une partie du circuit de gaz principal (200) et à permettre une circulation de gaz dans le circuit de purge (250), en réponse à un appui digital par l'utilisateur sur la touche virtuelle (7.1).

2. Appareil selon la revendication 1, **caractérisé en ce que** les premiers et seconds moyens de contrôle de flux (210 ; 252) comprennent au moins une vanne (Vhi1, Vhi2 ; Vhi3, Vhi4).

3. Appareil selon la revendication 1, **caractérisé en ce que** :
- la touche de lancement de purge (7.1) est configurée pour être activée par un appui ou contact du doigt par l'utilisateur sur une zone de l'écran tactile (4),
- l'écran tactile (4) est configuré pour détecter le contact du doigt de l'utilisateur sur la zone de l'écran tactile (4) correspondant à la touche de lancement de purge (7.1), lorsque la touche de lancement de purge (7.1) est activée, et transmettre un signal d'activation aux moyens de pilotage (205) et
- lesdits moyens de pilotage (205) sont configurés pour interpréter ce signal d'activation comme un signal de début de purge.

4. Appareil selon les revendications 1 ou 3, **caractérisé en ce que** les moyens de pilotage (205) commandent un début de purge en agissant sur les premiers et seconds moyens de contrôle de flux (210 ; 252), en réponse à l'appui digital sur la touche virtuelle (7.1) et/ou au signal d'activation.

5. Appareil selon la revendication 1, **caractérisé en ce que** :
- le circuit de gaz principal (200) comprend deux tronçons d'entrée (201) agencés en parallèles, chaque tronçon d'entrée (201) comprenant une entrée de NO (2), lesdits deux tronçons d'entrée (201) se raccordant fluidiquement l'un à l'autre en un site de jonction (202) du circuit de gaz principal (200) ; et
- le circuit de purge (250) comprend deux tronçons de jonction (251) agencés en parallèles, chaque tronçon de jonction (251) venant se raccorder (en 253) à l'un des tronçon d'entrée (201) du circuit de gaz principal (200), en amont des premiers moyens de contrôle de flux (210) agencés sur ledit circuit de gaz principal (200), et lesdits deux tronçons de jonction (251) se raccordant fluidiquement l'un à l'autre en un site de réunion (254) du circuit de purge (250).

6. Appareil selon la revendication 5, **caractérisé en ce que** des seconds moyens de contrôle de flux (252) sont agencés sur les deux tronçons de jonction (251).

7. Appareil selon les revendications 1 et 2, **caractérisé en ce qu'**en réponse à un appui de l'utilisateur sur la touche de lancement de purge virtuelle (7.1) s'affichant sur l'écran d'affichage tactile (4), les moyens de pilotage (205) sont configurés pour piloter les électrovannes principales et de purge (Vhi1, Vhi2 ; Vhi3, Vhi4) de manière :
- à stopper la circulation de gaz dans la partie du circuit de gaz principal (200) situées en aval des électrovannes principales (Vhi1, Vhi2) en fermant ou maintenant en position de fermeture, les deux électrovannes principales (Vhi1, Vhi2),
- à maintenir en position de fermeture l'une des électrovannes de purge (Vhi3, Vhi4) et
- et à permettre une circulation de gaz dans le circuit de purge (250) en passant l'autre des électrovannes de purge (Vhi3, Vhi4) en position d'ouverture pour autoriser une circulation du gaz dans le circuit de purge (250).

8. Appareil selon la revendication 1, **caractérisé en ce que** les moyens de pilotage (205) comprennent un microcontrôleur.

9. Appareil selon la revendication 1, **caractérisé en ce que** les premiers et seconds moyens de contrôle de flux (210 ; 252) comprennent des électrovannes pilotées (Vhi1, Vhi2 ; Vhi3, Vhi4) par les moyens de pilotage (205).

10. Installation (100) de fourniture d'un mélange gazeux contenant du NO à un patient comprenant :
- au moins un récipient de NO (10) contenant le gaz contenant du NO, de préférence deux récipients de NO (10) ; de préférence deux récipients de NO (10),
- l'appareil de délivrance de NO (1) selon l'une des revendications précédentes ; et
- au moins un conduit flexible (12) reliant fluidiquement ledit au moins un récipient de NO (10) à l'appareil de délivrance de NO (1), de préférence ledit au moins un récipient de NO (10) et/ou l'appareil de délivrance de NO (1) sont agencés sur un chariot de transport.

11. Installation (100) selon la revendication 10, **caractérisée en ce qu'**au moins un dispositif de détente (102) est agencé en aval dudit au moins un récipient de NO (10), ledit au moins un conduit flexible (12) étant accordé fluidiquement audit qu'au moins un dispositif de détente (102).

12. Installation selon la revendication 10, **caractérisée en ce qu'**elle comprend en outre un ventilateur médical (50) configuré pour fournir un flux de gaz respiratoire contenant de l'O₂ ; et un circuit respiratoire (20 ; 21) auquel sont raccordés fluidiquement le ventilateur médical (50) et l'appareil de délivrance de NO (1).

13. Installation selon la revendication 10, **caractérisée en ce que** chaque récipient de NO (10) contient un mélange gazeux NO/N₂ contenant entre 100 et 2000 ppmv de NO, le reste étant de l'azote (N₂) et/ou chaque dispositif de détente (102) est configuré pour réduire la pression du mélange gazeux NO/N₂ jusqu'à une pression de détente inférieure ou égale à 10 bars, typiquement entre 2 et 7 bars

14. Installation selon la revendication 1, **caractérisée en ce que** l'appareil de délivrance de NO (1) comprend deux entrées de NO (2) configurées pour être raccordées chacune à un conduit flexible (12) d'acheminement de mélange NO/N₂.

15. Méthode pour remplacer un récipient de NO vide (10) raccordée fluidiquement par un conduit flexible (12) au circuit principal (200) de gaz de l'appareil de délivrance de NO (1) selon l'une des revendications 1 à 9 et/ou d'une installation d'administration de gaz (100) selon l'une des revendications 10 à 14, par un récipient de NO plein (10), ledit conduit flexible (12) contenant du NO gazeux, typiquement un mélange NO/N₂, à une pression résiduelle supérieure 1 bar, typiquement une pression résiduelle de moins de 10 bars, dans laquelle :
a. on débute une procédure de purge dudit conduit flexible (12) par appui digital d'un utilisateur sur une touche de lancement de purge (7.1) virtuelle affichée sur l'écran d'affichage tactile (4) de l'appareil de délivrance de NO (1), de manière à générer un signal d'activation transmis aux moyens de pilotage (205) de l'appareil de délivrance de NO (1),
b. en réponse audit signal d'activation, les moyens de pilotage (205) de l'appareil de délivrance de NO (1) contrôlent les flux gazeux circulant dans le circuit principal (200) et le circuit de purge (250) de l'appareil de délivrance de NO (1), de manière à :
- stopper la circulation de gaz dans la partie du circuit de gaz principal (200) située en aval d'au moins une électrovanne principale (Vhi1, Vhi2) agencée sur ledit circuit de gaz principal (200) de l'appareil de délivrance de NO (1), en fermant ou maintenant en position de fermeture ladite au moins une électrovanne principale (Vhi1, Vhi2), et
- autoriser une circulation de gaz dans le circuit de purge (250) de l'appareil de délivrance de NO (1) en passant une électrovanne de purge (Vhi4, Vhi3) en position d'ouverture de manière à mettre la portion de circuit de gaz principal (200) reliée fluidiquement au conduit flexible (12) à purger, en communication fluidique avec l'atmosphère, de préférence via un orifice de purge (6), de manière évacuer vers l'atmosphère, au moins une partie du NO gazeux à la pression résiduelle présent dans le conduit flexible (12) à purger et dans ladite portion de circuit principal (200),
c. opérer une déconnection par l'utilisateur du conduit flexible (12) du récipient de NO vide (10) à remplacer et/ou d'un dispositif détendeur (102) agencé entre le conduit flexible (12) et le récipient de NO vide (10) à remplacer, et
d. opérer une connexion par l'utilisateur du conduit flexible (12) à un récipient de NO plein (10) et/ou au dispositif détendeur (102) agencé entre le conduit flexible (12) et ledit récipient de NO plein (10).

## Revendications modifiées

### Revendications modifiées conformément à la règle 137(2) CBE.

1. Appareil de délivrance de NO (1) pour fournir un gaz contenant du NO comprenant :
- un circuit de gaz principal (200) pour véhiculer le gaz contenant du NO entre au moins une entrée de NO (2) et au moins une sortie de NO (5),
- ladite au moins une entrée (2) de NO configurée pour permettre d'y raccorder fluidiquement un conduit flexible (12) de manière à alimenter le circuit de gaz principal (200) avec le gaz contenant du NO amené par ledit conduit flexible (12),
- des premiers moyens de contrôle de flux (210) agencés sur ledit circuit de gaz principal (200), configurés pour contrôler la circulation du flux de gaz contenant du NO dans ledit circuit de gaz principal (200),
- des moyens de pilotage (205) à microprocesseur,
- un circuit de purge (250) raccordé fluidiquement (en 253) au circuit de gaz principal (200) entre ladite au moins une entrée de NO (2) et les premiers moyens de contrôle de flux (210), ledit circuit de purge (250) comprenant une sortie de purge (6) en communication fluidique avec l'atmosphère, et
- des seconds moyens de contrôle de flux (252) agencés sur ledit circuit de purge (250), configurés pour contrôler la circulation de gaz dans ledit circuit de purge (250) en direction de la sortie de purge (6), et
- des moyens de déclenchement de purge (7), actionnables par l'utilisateur,
**caractérisé en ce que** :
- il comprend en outre un écran d'affichage tactile (4),
- les moyens de déclenchement de purge (7) comprennent une touche de lancement de purge virtuelle (7.1) s'affichant sur l'écran d'affichage tactile (4) et
- les moyens de pilotage (205) sont configurés pour piloter les premiers et seconds moyens de contrôle de flux (210 ; 252) de manière à stopper la circulation de gaz dans au moins une partie du circuit de gaz principal (200) et à permettre une circulation de gaz dans le circuit de purge (250), en réponse à un appui digital par l'utilisateur sur la touche virtuelle (7.1).

2. Appareil selon la revendication 1, **caractérisé en ce que** les premiers et seconds moyens de contrôle de flux (210 ; 252) comprennent des électrovannes (Vhi1, Vhi2 ; Vhi3, Vhi4) pilotées par les moyens de pilotage (205).

3. Appareil selon la revendication 1, **caractérisé en ce que** :
- la touche de lancement de purge (7.1) est configurée pour être activée par un appui ou contact du doigt par l'utilisateur sur une zone de l'écran tactile (4),
- l'écran tactile (4) est configuré pour détecter le contact du doigt de l'utilisateur sur la zone de l'écran tactile (4) correspondant à la touche de lancement de purge (7.1), lorsque la touche de lancement de purge (7.1) est activée, et transmettre un signal d'activation aux moyens de pilotage (205) et
- lesdits moyens de pilotage (205) sont configurés pour interpréter ce signal d'activation comme un signal de début de purge.

4. Appareil selon les revendications 1 ou 3, **caractérisé en ce que** les moyens de pilotage (205) commandent un début de purge en agissant sur les premiers et seconds moyens de contrôle de flux (210 ; 252), en réponse à l'appui digital sur la touche virtuelle (7.1) et/ou au signal d'activation.

5. Appareil selon la revendication 1, **caractérisé en ce que** :
- le circuit de gaz principal (200) comprend deux tronçons d'entrée (201) agencés en parallèles, chaque tronçon d'entrée (201) comprenant une entrée de NO (2), lesdits deux tronçons d'entrée (201) se raccordant fluidiquement l'un à l'autre en un site de jonction (202) du circuit de gaz principal (200) ; et
- le circuit de purge (250) comprend deux tronçons de jonction (251) agencés en parallèles, chaque tronçon de jonction (251) venant se raccorder (en 253) à l'un des tronçon d'entrée (201) du circuit de gaz principal (200), en amont des premiers moyens de contrôle de flux (210) agencés sur ledit circuit de gaz principal (200), et lesdits deux tronçons de jonction (251) se raccordant fluidiquement l'un à l'autre en un site de réunion (254) du circuit de purge (250).

6. Appareil selon les revendications 2 et 5, **caractérisé en ce que** les seconds moyens de contrôle de flux (252) comprennent des électrovannes de purge (Vhi3, Vhi4) agencées sur les deux tronçons de jonction (251).

7. Appareil selon les revendications 2 ou 5, **caractérisé en ce que** les premiers moyens de contrôle de flux (210) comprennent des électrovannes principales (Vhi1, Vhi2) pilotées par les moyens de pilotage (205).

8. Appareil selon les revendications 1, 2, 6 et 7, **caractérisé en ce qu'**en réponse à un appui de l'utilisateur sur la touche de lancement de purge virtuelle (7.1) s'affichant sur l'écran d'affichage tactile (4), les moyens de pilotage (205) sont configurés pour piloter les électrovannes principales et de purge (Vhi1, Vhi2 ; Vhi3, Vhi4) de manière :
- à stopper la circulation de gaz dans la partie du circuit de gaz principal (200) situées en aval des électrovannes principales (Vhi1, Vhi2) en fermant ou maintenant en position de fermeture, les deux électrovannes principales (Vhi1, Vhi2),
- à maintenir en position de fermeture l'une des électrovannes de purge (Vhi3, Vhi4) et
- et à permettre une circulation de gaz dans le circuit de purge (250) en passant l'autre des électrovannes de purge (Vhi3, Vhi4) en position d'ouverture pour autoriser une circulation du gaz dans le circuit de purge (250).

9. Appareil selon la revendication 1, **caractérisé en ce que** les moyens de pilotage (205) comprennent un microcontrôleur.

10. Installation (100) de fourniture d'un mélange gazeux contenant du NO à un patient comprenant :
- au moins un récipient de NO (10) contenant le gaz contenant du NO, de préférence deux récipients de NO (10) ;
- l'appareil de délivrance de NO (1) selon l'une des revendications précédentes ; et
- au moins un conduit flexible (12) reliant fluidiquement ledit au moins un récipient de NO (10) à l'appareil de délivrance de NO (1), de préférence ledit au moins un récipient de NO (10) et/ou l'appareil de délivrance de NO (1) sont agencés sur un chariot de transport.

11. Installation (100) selon la revendication 10, **caractérisée en ce qu'**au moins un dispositif de détente (102) est agencé en aval dudit au moins un récipient de NO (10), ledit au moins un conduit flexible (12) étant accordé fluidiquement audit qu'au moins un dispositif de détente (102).

12. Installation selon la revendication 10, **caractérisée en ce qu'**elle comprend en outre un ventilateur médical (50) configuré pour fournir un flux de gaz respiratoire contenant de l'O₂ ; et un circuit respiratoire (20 ; 21) auquel sont raccordés fluidiquement le ventilateur médical (50) et l'appareil de délivrance de NO (1).

13. Installation selon la revendication 11, **caractérisée en ce que** chaque récipient de NO (10) contient un mélange gazeux NO/N₂ contenant entre 100 et 2000 ppmv de NO, le reste étant de l'azote (N₂) et/ou chaque dispositif de détente (102) est configuré pour réduire la pression du mélange gazeux NO/N₂ jusqu'à une pression de détente inférieure ou égale à 10 bars, typiquement entre 2 et 7 bars

14. Installation selon la revendication 10, **caractérisée en ce que** l'appareil de délivrance de NO (1) comprend deux entrées de NO (2) configurées pour être raccordées chacune à un conduit flexible (12) d'acheminement de mélange NO/N₂.

15. Méthode pour remplacer un récipient de NO vide (10) raccordée fluidiquement par un conduit flexible (12) au circuit principal (200) de gaz de l'appareil de délivrance de NO (1) selon l'une des revendications 1 à 9 ou d'une installation d'administration de gaz (100) selon l'une des revendications 10 à 14, par un récipient de NO plein (10), ledit conduit flexible (12) contenant du NO gazeux, typiquement un mélange NO/N₂, à une pression résiduelle supérieure 1 bar, typiquement une pression résiduelle de moins de 10 bars, dans laquelle :
a. on débute une procédure de purge dudit conduit flexible (12) par appui digital d'un utilisateur sur une touche de lancement de purge (7.1) virtuelle affichée sur l'écran d'affichage tactile (4) de l'appareil de délivrance de NO (1), de manière à générer un signal d'activation transmis aux moyens de pilotage (205) de l'appareil de délivrance de NO (1),
b. en réponse audit signal d'activation, les moyens de pilotage (205) de l'appareil de délivrance de NO (1) contrôlent les flux gazeux circulant dans le circuit principal (200) et le circuit de purge (250) de l'appareil de délivrance de NO (1), de manière à :
- stopper la circulation de gaz dans la partie du circuit de gaz principal (200) située en aval d'au moins une électrovanne principale (Vhi1, Vhi2) agencée sur ledit circuit de gaz principal (200) de l'appareil de délivrance de NO (1), en fermant ou maintenant en position de fermeture ladite au moins une électrovanne principale (Vhi1, Vhi2), et
- autoriser une circulation de gaz dans le circuit de purge (250) de l'appareil de délivrance de NO (1) en passant une électrovanne de purge (Vhi4, Vhi3) en position d'ouverture de manière à mettre la portion de circuit de gaz principal (200) reliée fluidiquement au conduit flexible (12) à purger, en communication fluidique avec l'atmosphère, de préférence via un orifice de purge (6), de manière évacuer vers l'atmosphère, au moins une partie du NO gazeux à la pression résiduelle présent dans le conduit flexible (12) à purger et dans ladite portion de circuit principal (200),
c. opérer une déconnection par l'utilisateur du conduit flexible (12) du récipient de NO vide (10) à remplacer et/ou d'un dispositif détendeur (102) agencé entre le conduit flexible (12) et le récipient de NO vide (10) à remplacer, et
d. opérer une connexion par l'utilisateur du conduit flexible (12) à un récipient de NO plein (10) et/ou au dispositif détendeur (102) agencé entre le conduit flexible (12) et ledit récipient de NO plein (10).
